# EUROPEAN PATENT SPECIFICATION

(11) **EP 2 476 324 B1**
(45) Date of publication and mention of the grant of the patent: **29.07.2015**
(21) Application number: 09848999.0
(22) Date of filing: 06.09.2009
(51) Int. Cl.: A41B 9/04, A41D 27/06, A41D 31/00, A41D 27/02, A41D 27/24, A41D 31/02, A41B 9/12, A41B 17/00, A61F 13/06, B29C 65/08, B29C 65/62, B32B 5/26, B29L 31/48, A61F 13/66, B29C 65/50, B29C 65/00, B29C 65/48

(54) **WATERPROOF MOISTURE-PERMEABLE UNDERWEAR AND WATERPROOF MOISTURE-PERMEABLE LINER**
WASSERDICHTE FEUCHTIGKEITSDURCHLÄSSIGE UNTERWÄSCHE UND WASSERDICHTE FEUCHTIGKEITSDURCHLÄSSIGE AUSKLEIDUNG
SOUS-VÊTEMENT PERMÉABLE À L'HUMIDITÉ ET IMPERMÉABLE À L'EAU ET REVÊTEMENT PERMÉABLE À L'HUMIDITÉ ET IMPERMÉABLE À L'EAU

(43) Date of publication of application: 18.07.2012
(73) Proprietor: Komoriya, Noriko, Tokyo 181-0013 (JP)
(72) Inventor: Komoriya, Noriko, Tokyo 181-0013 (JP)
(74) Representative: Oficina Ponti, SLP
(86) International application number: PCT/JP2009/065540
(87) International publication number: WO 2011/027468

(56) References cited:
- JP-A- 11 124 710
- JP-A- 2001 020 102
- JP-A- 2002 013 001
- JP-A- 2005 530 641
- JP-U- 3 154 444

## Description

### TECHNICAL FIELD

The present invention relates to a waterproof moisture-permeable underwear to be worn on the lower body, and capable of preventing menstrual blood or urinary incontinence from leaking out to outer surface thereof, and avoiding wetness backside thereof.

### Background

Conventionally, underwear intended to prevent menstrual blood leakage or urinary incontinence leakage is constructed by using a fabric having waterproof property or a waterproofed fabric. However, such a fabric in general does not have moisture-permeability, and thus even if it can prevent blood or urine from leaking outside, its inner side may get wet due to vapor or the like and cause a wearer to feel uncomfortable.

To eliminate such uncomfortable feeing, for example, sanitary shorts using a fabric having waterproof permeability is proposed in Patent document 1.

In the disclosure, the "gusset" located in the crotch portion of the shorts is constructed in three layers, i.e., an inner layer, a middle layer, and an outer layer. The inner layer uses a material having stain-proof property and hygroscopic property. The middle layer uses a material having waterproof moisture-permeability (liquid impermeable and vapor permeable property). The outer layer uses a material that allows vapor to pass through well and has good looking.

Such construction enables to prevent menstrual blood or urine from leaking outside, and prevent the inside from getting wet, and to provide a good appearance.

### Prior art document

### Patent document

Patent document 1: microfilm of Japanese utility model application number 1980-138429 (Japanese utility model publication number 1982-60619).

Patent JP2002013001 A describes a trunks-type disposable pants having a space for covering a wearer's abdomen and buttocks therein. The trunks-type disposable pants comprises a pair of both side bodies and a center body positioned between the both sides bodies, wherein the both sides bodies each has crotch side parts and leg outside parts. The center body has a crotch front part, a crotch back part, a crotch part and a pair of leg inside parts, where the crotch part are respectively extended so as to cross over the crotch side parts. The leg inside parts are extended in parallel with the leg outside parts. The crotch side parts and the crotch front and back parts are respectively fixed at the remaining edge parts excluding upper edge parts forming a waist opening. The leg outside parts and the leg inside parts are respectively fixed at the remaining edge parts excluding bottom edge parts forming a leg circumference opening.

### Summary of the invention

### Problems to be solved by the invention

However, the sanitary shorts described above are worn directly on the skin, and thus a variety of functionalities, for example, stain-proof property, hygroscopicity, texture (soft feeling), and the like, are required. As a result, there has been a problem in that the scope of choices for a material for the inner layer is limited. In addition, since the product is shorts, it is necessary to take into account its external appearance to some extent, and thus the degree of choices for a material for the outer layer is also limited.

For the underwear of this type for which greater importance is placed on waterproofness, even if the material of the fabric piece itself has high waterproofness, menstrual blood or urine may leak from junction areas (connections) between the fabric pieces. It is therefore essential to enhance waterproofness in the junction areas.

In a case where the sanitary shorts as described above are worn, even if the wearer has high confidence in the waterproofness of the sanitary shorts, the wearer is plagued by concerns, for example, that a larger amount of menstrual blood or a greater amount of wearer's movements may possibly cause the blood to leak out of the sanitary shorts and thus an outer garment such as a skirt or trousers may be stained with them. Such mental concerns hang over the wearer's head, and may place a great constraint on activities in business or leisure of the wearer.

Although the problems in sanitary shorts are presented in the foregoing description, there are almost similar problems also in underwear for incontinence.

The present invention therefore aims to provide a waterproof moisture-permeable underwear by which the scope of choices for a material can be widened, and the waterproofness in the junction areas can be improved so that staining of an outer garment with menstrual blood or urine leaked from undergarment can be surely prevented.

### Measures for solving the problems

The invention according to claim 1 relates to a waterproof moisture-permeable underwear to be worn between undergarment and an outer garment worn on the lower body. The waterproof moisture-permeable underwear according to the invention is characterized in that the underwear is constructed by joining a plurality of two-layered fabric pieces, each of which comprises a base fabric disposed such that it faces the undergarment, and a membrane laminated outside of the base fabric and having waterproof moisture-permeability, in junction areas. A material superior to the membrane in texture is selected for the base fabric, and a material superior to the base fabric in welding property is selected for the membrane, and at least one of the junction areas is formed by welding.

The invention according to claim 2 is characterized in that the junction area formed by welding in the waterproof moisture-permeable underwear according to claim 1 is formed by welding in such a condition that respective junction allowances of the fabric pieces being adjacent to each other are folded inward and the membranes in the respective junction allowances are brought into contact with each other.

The invention according to claim 3 is characterized in that the junction area formed by welding in the waterproof moisture-permeable underwear according to claim 1 is formed by welding in such a condition that respective junction allowances of the fabric pieces being adjacent to each other are extended outward and the base fabrics in the respective junction allowances are brought into contact with each other.

The invention according to claim 4 is characterized in that the junction area formed by welding in the waterproof moisture-permeable underwear according to claim 1 is formed by welding in such a condition that one junction allowance of respective junction allowances of the fabric pieces being adjacent to each other is overlaid on the other junction allowance such that the membrane in the one junction allowance is brought into contact with the base fabric in the other junction allowance.

The invention according to claim 5 is characterized in that the junction area formed by welding in the waterproof moisture-permeable underwear according to claim 1 is formed by welding in such a condition that respective junction allowances of the fabric pieces being adjacent to each other are folded inward and the membranes in the respective junction allowances are brought into contact with each other, or in such a condition that respective junction allowances of the fabric pieces being adjacent to each other are extended outward and the base fabrics in the respective junction allowances are brought into contact with each other. When the fabric pieces being joined in the junction area are joined in another junction area that intersects with these junction areas in such a condition that one of the junction areas and the other junction area continuously converge each other, the one junction area is joined to the another junction area with its edge portion being folded in a direction, and the other junction area is joined to the another junction area with its edge portion being folded in a direction opposite to that of the one junction area.

The invention according to claim 6 is characterized in that the junction area formed by welding in the waterproof moisture-permeable underwear according to claim 1 is formed by aligning respective end edges of the fabric pieces being adjacent to each other and welding a band of sealing material on the end edges.

The invention according to claim 7 is characterized in that the junction area of the plurality of fabric pieces to be welded in the waterproof moisture-permeable underwear according to claim 1 includes a crotch line and a crotch portion.

The invention according to claim 8 is characterized in that the fabric piece in the waterproof moisture-permeable underwear according to claim 1 has a degree of stretch in one direction and a different degree of stretch in another direction orthogonal to the one direction, and the fabric piece is disposed such that a direction having a higher degree of stretch is horizontal.

### Effects of the invention

According to the invention of claim 1, the fabric piece of the waterproof moisture-permeable underwear is constructed by a laminate of a base fabric and a membrane having waterproof moisture-permeability. Therefore, even when menstrual blood or urine penetrates through and leaks out of the undergarment (for example, shorts, sanitary shorts, briefs) worn inside the waterproof moisture-permeable underwear, staining of an outer garment (for example, a skirt, trousers) worn outside of the waterproof moisture-permeable underwear with the urine or menstrual blood can be surely prevented. In addition, the underwear allows vapor or the like inside the underwear to pass through to outside, and prevents the inside from getting wet.

In addition, an outer garment is worn outside of the waterproof moisture-permeable underwear, unlike the case used for such an outer garment as a raincoat. Thus, the membrane itself is covered by the outer garment even if the membrane is disposed outside of the base fabric. Therefore, it is not necessary to provide a specific layer for protecting the surface of the membrane or a specific layer for making the membrane look good. As such, it is possible to construct the underwear by lightweight, two-layered fabric pieces each of which comprises a base fabric and a membrane laminated thereon.

Furthermore, the scope of choices for a material for the base fabric disposed inside is wider than that of the membrane, and thus the feeling when worn can be improved by selecting a material of nice and soft texture for the base fabric. The waterproof moisture-permeable underwear is worn on undergarment. However, depending on the size or shape of the undergarment, the waterproof moisture-permeable underwear may directly contact with the skin, and therefore it is desirable that the base fabric, which is disposed inner side of the underwear, have good texture.

In addition, by using welding to form the junction area of the fabric pieces, the leakage of menstrual blood or urine from the junction area can be surely prevented.

According to the invention of claim 2, respective junction allowances of the fabric pieces being adjacent to each other are folded inward and welded in such a condition that the membranes having superior welding property are brought into contact with each other, and thus good welding can be performed. In addition, the junction area is not exposed when worn and thus a good-looking can be obtained.

According to the invention of claim 3, respective junction allowances of the fabric pieces being adjacent to each other are extended outward and can be welded in such a condition that the base fabrics are brought into contact with each other.

According to the invention of claim 4, respective junction allowances of the fabric pieces being adjacent to each other can be welded in such a condition that one junction allowance is overlaid on the other junction allowance and the base fabric is brought into contact with the membrane.

According to the invention of claim 5, rough feeling can be reduced compared with a case the edge portion of one junction area and the edge portion of the other junction area are folded in the same direction when welded to another junction area.

According to the invention of claim 6, the thickness of the junction area is the sum of the thicknesses of one fabric and one sealing material only, and thus poor texture due to thicker thickness can be avoided.

According to the invention of claim 7, the leakage of menstrual blood or urine can be surely prevented by welding a crotch line or a crotch portion, since the menstrual blood or urine is likely to leak at these portions.

According to the invention of claim 8, the fabric piece has a higher degree of stretch in horizontal direction, and thus the waterproof moisture-permeable underwear can be easily worn and taken off, and the fit feeling or the mobility when worn can be improved.

### Brief description of drawings

Figs. 1A and 1B illustrate a first embodiment. Fig. 1A is an oblique view of underwear 11 viewed from front and obliquely above. Fig. 1B is an exploded oblique view of the underwear 11.
Fig. 2A is an oblique view illustrating the layer structure and the stretch of a fabric piece (cloth) A. Fig. 2B illustrates that the moisture-permeability of the fabric piece A varies depending on temperatures.

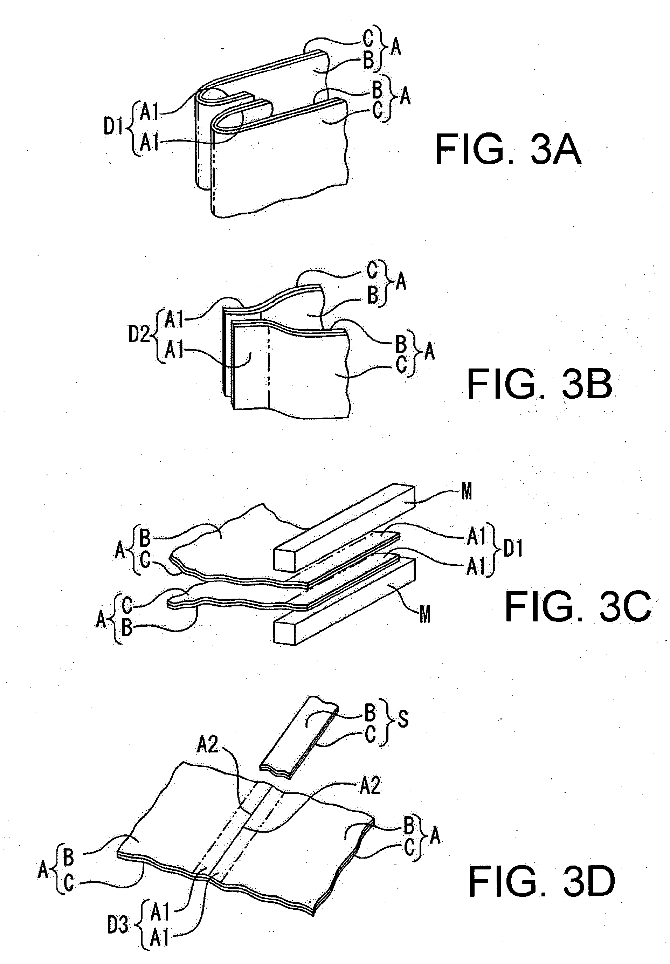

Fig. 3A is an oblique view illustrating an example in which a junction area D1 is formed by folding junction allowances A1, A1 at edge portions of two fabric pieces A, A inward and joining them. Fig. 3B is an oblique view illustrating an example in which a junction area D2 is formed by extending junction allowances A1, A1 at edge portions of two fabric pieces A, A outward and joining them. Fig. 3C is an oblique view illustrating that two fabric pieces A, A are welded. Fig. 3D is an oblique view illustrating that junction allowances A1, A1 of two fabric pieces A, A are welded using a seam tape (sealing material) S.
Figs. 4A and 4B illustrate a second embodiment. Fig. 4A is a view of underwear 21 viewed from front and obliquely above. Fig. 4B illustrates the underwear 21 of Fig. 4A with a portion thereof being cutaway.
Fig. 5A is a view taken along an arrowed line V-V in Fig. 4A. Fig. 5B illustrates the joint condition of a portion at which a lower edge of a junction area 21A and a lower edge of a junction area 21B intersect with junction areas 21E, 21F, viewed from inside.
Figs. 6A and 6B illustrate a third embodiment. Fig. 6A is a view of underwear 31 viewed from front and obliquely above. Fig. 6B is a view taken along an arrowed line B-B in Fig. 6A.
Figs. 7A and 7B illustrate a fourth embodiment. Fig. 7A is an oblique view of a skirt 42 viewed from front and obliquely above. Fig. 7B is an exploded oblique view in which a skirt body 43 and a liner 41 are separated.
Figs. 8A-8C illustrate a fifth embodiment. Fig. 8A is an oblique view of short pants 52 viewed from front and obliquely above, Fig. 8B is an oblique view of a liner 51 viewed from front and obliquely above. Fig. 8C is an oblique view of a short pants body 53 viewed from front and obliquely above.

### Embodiments for implementing the invention

Embodiments of the present invention are described below in detail with reference to the drawings. In each drawing, same reference symbols are given for materials or components having a same structure, and duplicate explanation thereof is omitted. In addition, in each drawing, components that are not required for the explanation are omitted as appropriate. In some drawings, directions, up, down, right, and left are shown in arrows. These directions, up, down, right, and left refer to the directions of up, down, right, and left of waterproof moisture-permeability underwear 10, a waterproof moisture-permeability liner 40, etc. viewed from a wearer.

Although the waterproof moisture-permeable underwear 10 to which the present invention is applied is called underwear, the description herein is based on the premise that another undergarment for preventing menstrual blood leakage or urine leakage, for example, sanitary shorts or underwear for incontinence, or the like, is worn underneath (inside). In other words, the waterproof moisture-permeable underwear 10 is intended, when the menstrual blood or urine penetrates through and leaks out of the undergarment worn inside the underwear 10, to prevent staining of the outer garment such as a skirt or trousers worn on (outside) the waterproof moisture-permeable underwear 10 with the menstrual blood or urine. For this reason, it is not required taking into consideration the external appearance of the waterproof moisture-permeable underwear 10 so much, and greater importance is placed on its functionality.

Similar consideration is applied to the waterproof moisture-permeable liner 40 to which the present invention is applied.

### <First embodiment>

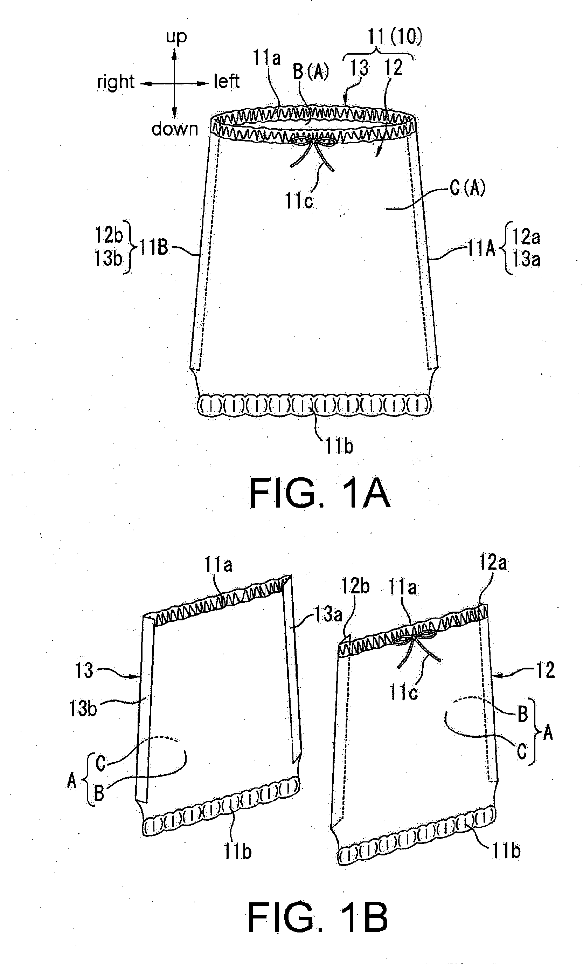

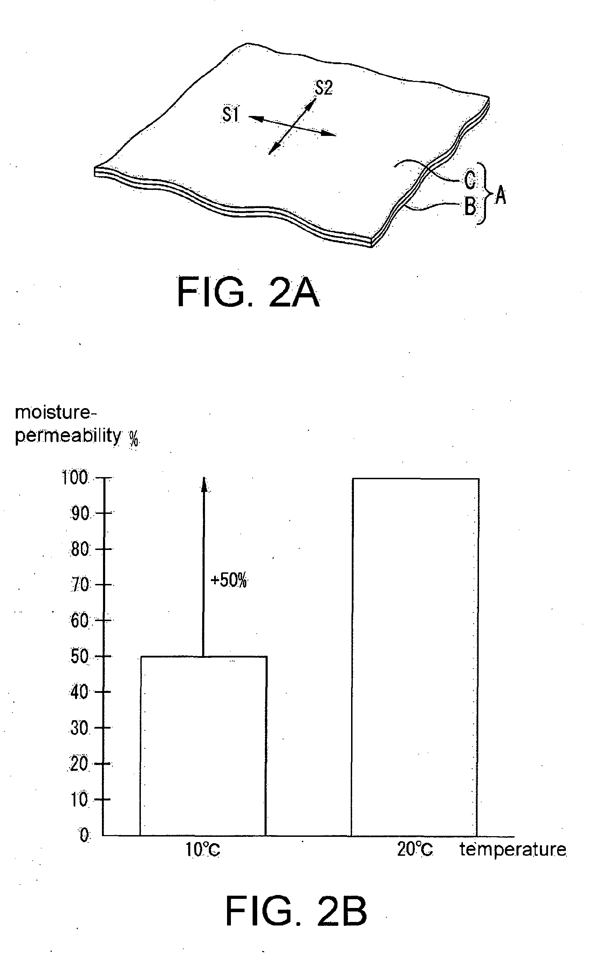

An example in which the waterproof moisture-permeable underwear 10 is skirt-like underwear 11 is described, with reference to Fig. 1A to Fig. 3D. Fig. 1A is an oblique view of the underwear 11 viewed from front and obliquely above, and Fig. 1B is an exploded oblique view of the underwear 11. Fig. 2A is an oblique view illustrating the layer structure and the stretch of a fabric piece (cloth) A, and Fig. 2B illustrates that the moisture-permeability of the fabric piece A varies depending on temperatures. Fig. 3A is an oblique view illustrating an example in which a junction area D1 is formed by folding the junction allowances A1, A1 at edge portions of two fabric pieces A, A inward and joining them. Fig. 3B is an oblique view illustrating an example in which a junction area D2 is formed by extending the junction allowances A1, A1 outward at edge portions of two fabric pieces A, A outward and joining them. Fig. 3C is an oblique view illustrating that two fabric pieces A, A are welded. Fig. 3D is an oblique view illustrating the junction allowances A1, A1 of two fabric pieces A, A are welded by a seam tape (sealing material) S.

As shown in Figs. 1A, 1B, the underwear 11 is formed in a roughly truncated cone, skirt-like shape by joining two fabric pieces A, A each cut into a predetermined shape, in other words, a front panel 12 and a back panel 13, in a junction area 11A on the left side and in a junction area 11B on the right side.

The front panel 12 is formed into a roughly trapezoid, and long bands of junction allowances 12a, 12b each folded inward are provided roughly vertically at both edge portions on the right and left thereof. The back panel 13 is also formed similarly into a roughly trapezoid, and long bands of junction allowances 13a, 13b each folded inward are provided roughly vertically at both edge portions on the right and left thereof. The front panel 12 and the back panel 13 are shaped into the skirt-like underwear 11 by constructing integrally such that the junction allowance 12a, 13a on the left side are joined together to form the junction area 11A, and the junction allowance 12b, 13b on the right side are joined together to form the junction area 11B. Under this condition, the upper edge of the underwear 11 is formed annularly, and, for example, elastic 11a may be sewn at the upper edge along the periphery in order to prevent the underwear 11 from slipping down when worn. The lower edge of the underwear 11 is also formed annularly, and decorative lace 11b is provided at the lower edge roughly along the periphery. In the illustrated example, a decorative ribbon 11 c is attached at a front upper edge of the underwear 11.

Portions other than the elastic 11a, the lace 11b, the ribbon 11c, etc. of the skirt-like underwear 11 constructed as described above are formed of the fabric piece A having waterproof moisture-permeability. The fabric piece A having waterproof moisture-permeability is a fabric that does not allow liquid to pass through, but allows vapor to pass through. As the fabric piece A having such waterproof moisture-permeability, for example, GORE-TEX (Registered trademark) Fabrics manufactured by Japan Gore-Tex Inc., OMNI-TECH manufactured by Columbia Sportswear Company, DRYTEC (registered trademark) manufactured by MontBel Co. Ltd., c_change (registered trademark) from Schoeller, etc are known.

Among these materials, c_change is superior in (1) waterproof moisture-permeability, and (2) by which lightweight can be achieved, and has (3) good texture (soft feeling), (4) stretch, (5) breathability, windproof property, and (6) good welding property. Having such functionalities, c_change can be preferably used as the fabric piece A for making the underwear 11 of this embodiment, the underwear 21 of the second embodiment, the underwear 31 of the third embodiment, the liner 41 of the fourth embodiment, the liner 51 of the fifth embodiment. The description hereinafter is explained on the premise that the fabric piece A is c_change, however, regarding waterproof moisture-permeability, other than c_change, for example, GORE-TEX (Registered trademark) Fabrics or the like may also be used as the fabric piece A, of course.

### (1) Waterproof moisture-permeability

The fabric piece A is constructed by a base fabric (base) B and a membrane (thin film) C as shown in Fig. 2A. In other words, the fabric piece A is a two-layered (two layer structure) fabric piece constructed by laminating the membrane C having waterproof moisture-permeability on one surface of the base fabric B.

The fabric piece A contains 30 wt% nylon (base fabric B), and 70 wt% polyurethane (membrane C). By adopting such constituents, the fabric piece A achieves waterproof moisture-permeability. As long as the base fabric B surely supports the membrane C and does not disturb the waterproof moisture-permeability of the membrane C, a synthetic resin other than nylon, or even cotton or the like can also be used as the base fabric B.

### (2) Lightweight

Since the underwear 11 in this embodiment uses two-layered fabric pieces A, lightweight can be sought compared with those that use general three-layered fabric pieces. A typical example for a garment constructed by a waterproof moisture-permeable material is a raincoat. If the membrane of a raincoat is damaged or peeled, it may allow rain water to enter therefrom. To protect the membrane, therefore, a protecting layer is provided outside the membrane. In addition, a layer for improving the texture (soft feeling), for example, is provided inside the membrane. In other words, a raincoat in general is formed in three layers, and thus the weight tends to be added. There is a two-layered raincoat that does not have an inner layer, however, in this case, a special treatment is provided on the backside of the membrane for improving the texture, and the treatment may increase the cost for creating the fabric.

In contrast, since it is the premise that an outer garment is worn on the underwear 11, the membrane C can be disposed outer side of the fabric piece A, and the base fabric B can be disposed inner side of the fabric piece A. As such, a two-layered construction can be achieved, and thus lightweight can be more readily achieved than with a three-layered construction. In addition, there is no need to provide a special treatment on the backside of the membrane, and thus the underwear can be constructed at lower cost.

### (3) Texture (soft feeling)

In addition, the underwear 11 has a wider scope of choices for a material for the base fabric B. Although the underwear 11 is called underwear, the description herein is based on the premise that undergarment is worn inside. Depending on the size or shape of the undergarment, a portion of the base fabric B that extends over the undergarment may directly contact with the skin of the wearer. Since the base fabric B may contact with the skin as such, a good texture (soft feeling) is required to some extent but not as much as required for the undergarment, and the scope of choices for the material is widened accordingly. As a result, for example, a texture as good as the texture required for the undergarment is not required, and other functionalities can be enhanced (for example, achieving lightweight or improving breathability) accordingly. In this embodiment, a material superior in texture to at least the membrane C is adopted for the base fabric B. More specifically, a synthetic resin other than nylon can be also used for the base fabric B on the condition that the base fabric B is superior to the membrane C in texture.

### (4) Stretch

In addition, the fabric piece A has stretch, and, as shown in Fig. 2A, for example, the degree of stretch in direction S 1 (one direction) is higher than the degree of stretch in the direction S2 (other direction) that is orthogonal to the direction S1. Therefore in a case where the skirt-like underwear 11 as shown in Fig. 1A is formed by the fabric pieces A having such stretch, it is desirable that the direction S1 having a higher degree of stretch be placed horizontally (left-right direction) when the front panel 12 and the back panel 13 are cut from the fabric piece A. This enables the underwear 11 to be easily worn and taken off, and the fit feeling or the mobility can be improved when worn.

### (5) Breathability, windproof property

Fig. 2B illustrates experimental data on the relation between the temperature of the membrane C and moisture-permeability in a case the fabric piece A is c_change. In Fig. 2B, the horizontal axis shows temperatures (degrees centigrade), and the vertical axis shows moisture-permeability (%).

As temperature rises, the polymer structure of the membrane C opens discretely and thus moisture-permeability is improved. This facilitates to allow moisture to escape to outside, and also facilitates to allow excess heat emitted from the body to escape to outside. In contrast, as temperature lowers, the polymer structure of the membrane C tightly aligns and stores heat, and thus heat retention and comfortable moisture-permeability are maintained.

More specifically, it is found that, assuming that the moisture-permeability of the membrane C at a temperature of 20 degrees centigrade is 100%, the moisture-permeability at 10 degrees centigrade becomes 50%, as shown in Fig. 2B. (Source: Empa Test Report No. 841192-2 dated on Dec. 15, 2006). It is also found that the moisture-permeability monotonously increases as temperature rises and monotonously decreases as temperature lowers, in the range between the temperature of 10 degrees centigrade and 20 degrees centigrade.

In addition, the membrane C has breathability but still has windproof property. When wind strikes the membrane C, it bounces off air molecules. However, in a case where the static pressure differs between inside and outside of the membrane C, for example, in a case where when the inner temperature rises and the air pressure that includes partial pressure of vapor becomes higher than the outside air pressure, the air (vapor) can escape from inside to outside.

### (6) Welding property

As described above, the fabric piece A is constructed by the base fabric B and the membrane C, and polyurethane is adopted for the material of the membrane C. Polyurethane has thermoplastic property, and melts when heated and cures when cooled. Therefore, the fabric piece A can be welded well in such a condition that the membranes C, C made of polyurethane are brought into contact with each other, without sewing that uses a string. There are various types of welding, for example, high frequency welding in which the action of high frequency energy causes resin material itself to generate heat from inside, heat welding that heats by the action of thermal conductivity, ultrasonic welding in which ultrasonic energy causes a target material to vibrate and generate a strong frictional heat, and the like. For this embodiment, ultrasonic welding is preferable.

The junction of two adjacent fabric pieces A, A is described regarding the welding property, with reference to Figs. 3A-3D. The welding can be similarly used for a case in which one fabric piece A is looped and then a junction area of one edge portion thereof and a junction area of the other edge portion thereof are joined. Therefore, the case in which two fabric pieces A, A are joined is described below.

Figs. 3A and 3B illustrate examples in which two fabric pieces A, A are directly joined, and Fig. 3D illustrates two fabric pieces A, A are joined by using a seam tape (sealing material).

In Fig. 3A, the edge portion of each of the fabric pieces A, A are folded inward to form bands of junction allowances A1, A1 respectively, and these junction allowances A1, A1 are aligned each other and joined to form a junction area D1. In this case, joining may be done by using a general sewing that uses a string or by the welding shown in Fig. 3C. The welding can be performed by using, for example, an ultrasonic sewing machine. The two fabric pieces A, A are overlaid each other so that their membranes C, C face each other, and the junction areas A1, A1 at their edge portions are inserted between a pair of metal bars M, M. Then, ultrasonic wave is applied to the metal bars M, M. This activates the activities of urethane molecules in polyurethane that makes up the membrane C, and adhesion on the molecular level can be achieved. In the junction area D1 shown in Fig. 3A, the membranes (polyurethane) C, C are welded each other, and thus relatively good welding can be achieved compared with the case the membrane (polyurethane) C and the base fabric (nylon) B are welded, or the base fabrics (nylon) B, B are welded.

In Fig. 3B, bands of the junction allowances A1, A1 at the edge portion of each of the fabric pieces A, A are extended outward, and these junction areas A1, A1 are aligned each other and joined to form a junction area D2. The junction area D2 in this case may be formed either by a general sewing that uses a string or the welding shown in Fig. 3C. If welding is used, the welding is performed in such a condition that both of the fabric pieces A, A are inside out, in Fig. 3C. In this case, the base fabrics B, B are joined each other. The base fabrics B, B made of nylon have a poorer welding property than the membranes C, C made of polyurethane have, but polyurethane seeps out from the base fabric B, B when welded and achieves the welding. Required waterproofness can be sufficiently obtained even in a case, for example, the base fabrics B, B are welded each other by using an ultrasonic sewing machine as long as the conditions for welding are set as appropriate.

Other than the examples in Figs. 3A and 3B, there is another example in which the fabric pieces A, A are directly joined. One example is that one junction allowance A1 of one fabric piece A is simply overlaid on the other junction allowance A1 of the other fabric piece A to form a junction area and then the junction area is welded, and such a junction area may also be adopted. This junction area can be adopted when the junction allowances A1, A1 are not curved but linear, and can reduce fabrication cost compared with those in Figs. 3A and 3B. In this case, the junction area is welded in such a condition that the membrane C of one junction allowance A1 is brought into contact with the base fabric B of the other junction allowance A1. The junction strength of the junction area formed in this manner is smaller than the junction strength of the junction area D1 shown in Fig. 3A but greater than the junction strength shown in Fig. 3B.

Fig. 3D shows that a junction area D3 is formed by using a seam tape S. End edges A2, A2 of two fabric pieces A, A are aligned each other to form the junction allowances A1, A1, and then a band of the seam tape S is welded on the junction allowances A1, A1. A material similar to the fabric piece A, in other words, the base fabric B and the membrane C laminated thereon, is used for the seam tape S. The base fabrics B, B disposed inner side of the fabric pieces A, A are joined from inside such that with which the membrane C of the seam tape S contacts. This enables the seam tape S to be hidden inside the fabric pieces A, A and not exposed to outside, and thus good-looking can be achieved. In addition, since the base fabrics B, B of the fabric pieces A, A and the membrane C of the seam tape S are joined, the junction strength becomes greater than the junction area D2 in Fig. 3B in which the base fabrics B, B are joined each other. In addition, by using a material similar to the fabric piece A for the seam tape S, superior functionality the fabric piece A has can be achieved similarly also in the junction area D3 that includes the seam tape S. A material different from the fabric piece A can also be used for the seam tape. In this case, for example, a material having no waterproof moisture-permeability but having superior welding property, high degree of stretch, being thin, and having a greater strength or the like can be selected.

For the seam tape S, a seam tape to which hot-melt (adhesive that can be heat welding) is applied can also be used. In this case, two fabric pieces A, A can be joined by heating the hot-melt to melt it and cooling it to cure it.

The junction strength of the junction welded as described above is now described.

In terms of junction strength, the junction area D1 in Fig. 3A is strongest, and the junction area D3 in Fig. 3D is stronger than the junction area D2 in Fig. 3B. This is because the welding property of the membrane C being made of polyurethane is better than the welding property of the base fabric B made of nylon. Specifically, the membranes (polyurethane) C, C are joined in the junction area D1 in Fig. 3A, and the base fabric (nylon) B and the membrane (polyurethane) C are joined in the junction area D3 in Fig. 3D, and the base fabrics (nylon) B, B are joined the junction area D2 in Fig. 3B.

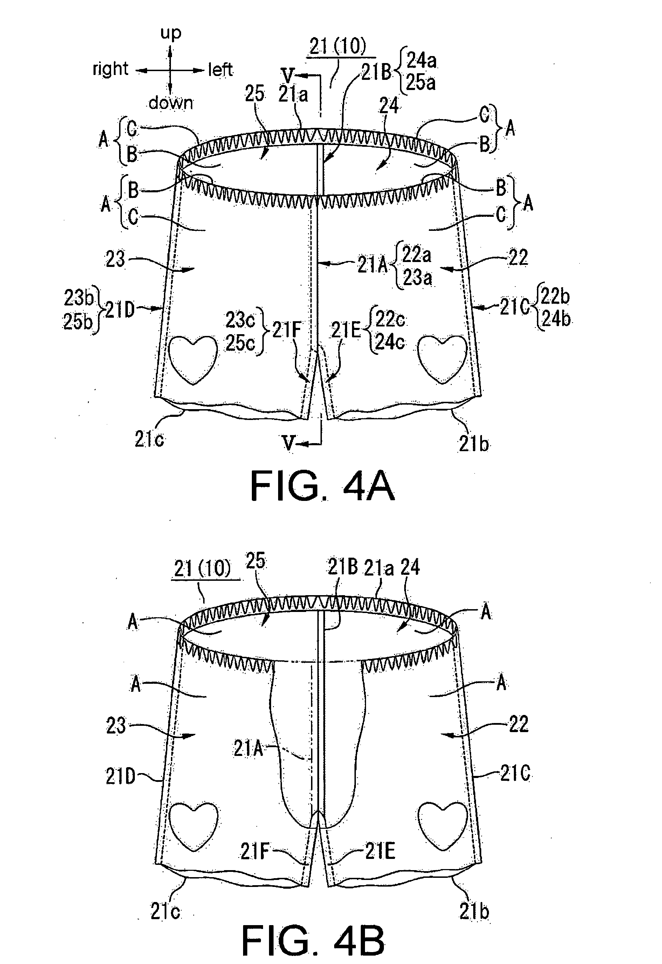

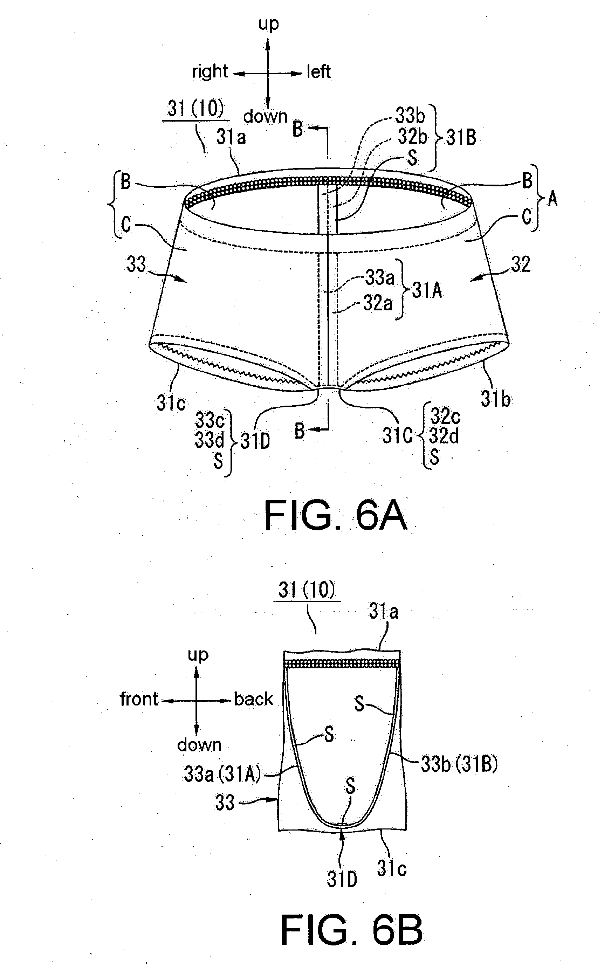

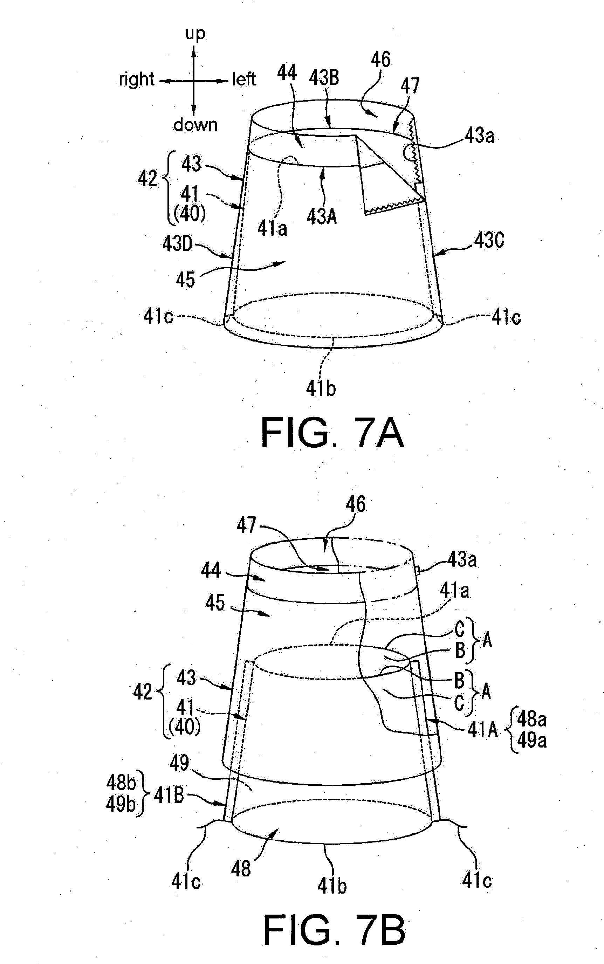

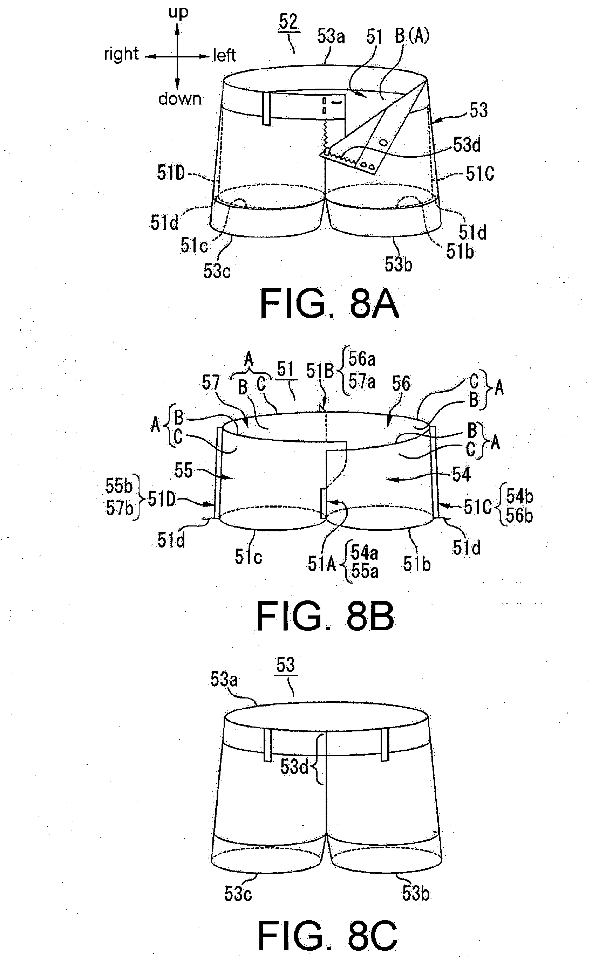

In terms of appearance, it is desirable that the junction area D1 in Fig. 3A be applied to the underwear 21 (see Figs. 4A, 4B), 31(see Figs. 6A, 6B), and the junction area D2 in Fig. 3B be used for the liner 41 (see Figs. 7A, 7B), 51 (see Figs. 8A-8C). Since the junction area D1 in Fig. 3A is folded inward, the area is not seen from outside when used for the underwear 21, etc. On the other hand, since the junction area D2 in Fig. 3B is extended outward, the area is not seen from either outside or inside when used for liner 41, etc. The junction area D3 in Fig. 3D can be alternatively used instead of the junction areas D1 in Fig. 3A, and D2 in Fig. 3B.

In terms of texture (soft feeling), the junction area D3 in Fig. 3D is better than the junction areas D1 in Fig. 3A and D2 in Fig. 3B. In other words, the thickness of the junction areas D1, D2 themselves is two times of the thickness of one fabric piece A, but in actual use they are folded on either one of the fabric pieces A and the thickness becomes substantially three times the thickness of one fabric piece A, and thus the rough feeling will be increased. On the other hand, since the junction area D3 in Fig. 3D uses the seam tape S made of a material similar to the fabric piece A, the thickness thereof is almost same as two times of one fabric piece A, and thus the rough feeling can be reduced compared with the three-times thickness.

In the underwear 11 shown in Figs. 1A, 1B, the junction area D1 shown in Fig. 3A is adopted for the junction areas 11A, 11B. As shown in Figs. 1A, 1B, the junction areas 11A, 11B of the underwear 11 is located on the left side and the right side of the underwear 11. Such locations are the portions that are not likely to contact with the menstrual blood or urine that penetrates through undergarment (not shown), for example, compared with a case in which the junction area is located in a front portion or back potion of the underwear 11. Therefore, in the underwear 11 shown in Figs. 1A, 1B, the junction areas 11A, 11B may be formed by a general sewing that uses a string, instead of welding. For example, in the underwear 11, 21, 31 or liners 41, 51, if a junction area is located near a portion from the crotch to the buttocks of a wearer, or near those portions, i.e., the portions at which menstrual blood or urine can leak, it is required the junction area should be welded in order to ensure waterproofness. However, for other junction areas, not only welding but also sewing that uses a string can be adopted. In a case where sewing is adopted, the junction allowance A1 would be a seam allowance.

As described above, the skirt-like underwear 11 as the waterproof moisture-permeable underwear 10 can prevent staining of the outer garment with menstrual blood or urine leaked from the undergarment by using the fabric piece A having waterproof moisture-permeability. In addition, by using the fabric piece A such that the base fabric B is disposed inner side and the membrane C is disposed outer side, two-layered construction being lightweight but having good texture (soft feeling) can be achieved. In addition, if a material having stretch, breathability, and windproof property is used for the fabric piece A, the underwear 11 can be smoothly worn and taken off, and can achieve a good fit, prevent wind entrance, and reduce wetness inside. In addition, by using a material having a good welding property for the membrane C, the welding of the junction area of the fabric piece A can ensure the waterproofness.

### <Second embodiment>

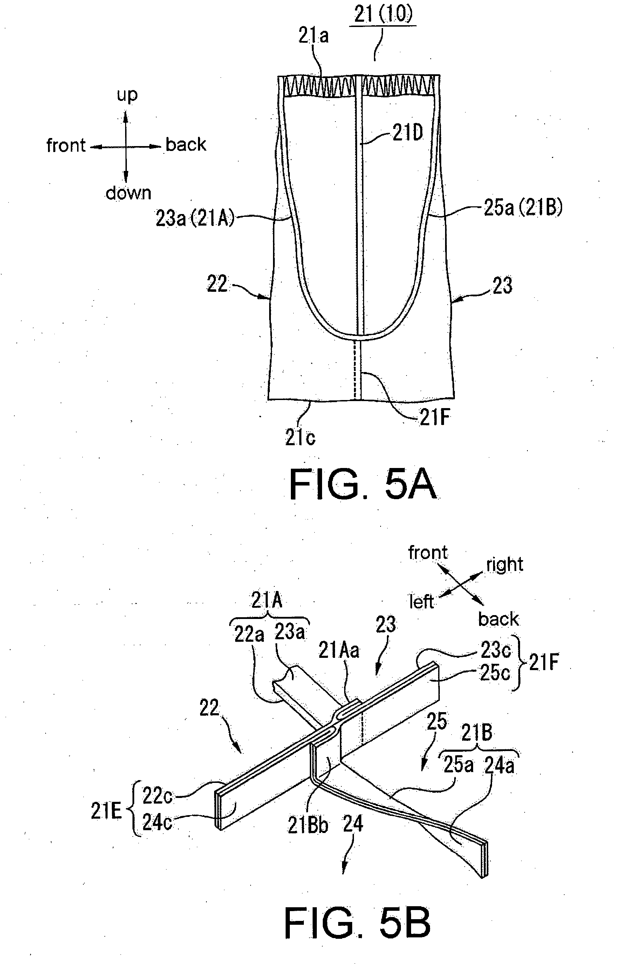

An example in which the waterproof moisture-permeable underwear 10 is the pants-like underwear 21 is illustrated, with reference to Figs. 4A, 4B and Figs. 5A, 5B. Fig. 4A is a view of the underwear 21 viewed from front and obliquely above. Fig. 4B illustrates the underwear 21 of Fig. 4A with a portion thereof being cutaway. Fig. 5A is a view taken along an arrowed line V-V in Fig. 4A. Fig. 5B illustrates the joint condition of a portion at which the lower edge of the junction area 21A and the lower edge of the junction area 21B intersect with the junction areas 21E, 21F, viewed from inside.

The underwear 21 of this embodiment is intended to be worn relatively (compared with underwear 31 of a third embodiment described later) loosely (in a loose and ease condition).

As shown in Figs. 4A, 4B, the underwear 21 is constructed by joining four fabric pieces A, A, ..., each cut into a predetermined shape, in other words, a left front panel 22, a right front panel 23, a left back panel 24, and a right back panel 25 in the junction areas 21A-21F.

The junction area 21A is located at the front center vertically in which a junction allowance (crotch line) 22a of the left front panel 22 and a junction allowance (crotch line) 23a of the right front panel 23 are joined. The junction area 21B is located at the back center vertically in which a junction allowance (crotch line) 24a of the left back panel 24 and a junction allowance (crotch line) 25a of the right back panel 25 are joined. The junction area 21C is located on the left side vertically in which a junction allowance 22b of the left front panel 22 and a junction allowance 24b of the left back panel 24 are joined. The junction area 21D is located on the right side vertically in which a junction allowance 23b of the right front panel 23 and a junction allowance 25b of the right back panel 25 are joined. The junction area 21E is extending from the lower end of the junction area 21A obliquely left downward in which a junction allowance (crotch portion) 22c of the left front panel 22 and a junction allowance (crotch portion) 24c of the left back panel 24 are joined. The junction area 21F is extending from the lower end of the junction area 21A right obliquely downward in which a junction allowance (crotch portion) 23c of the right front panel 23 and a junction allowance 25c of the right back panel 25 are joined. The junction area 21E and the junction area 21F are formed continuously.

By joining as described above, an upper edge 21a of the left front panel 22, the right front panel 23, the left back panel 24, and the right back panel 25 of the underwear 21 is linked annularly, and elastic (not shown) is sewn along the periphery of the upper edge 21a. This elastic of the underwear 21 enables a wearer to easily wear and take off it, and prevents slipping down when worn. In addition, a lower edge 21b of the left front panel 22 and the left back panel 24 is linked annularly to form an opening through which the left leg is inserted. Similarly, a lower edge 21c of the right front panel 23 and the right back panel 25 is linked annularly to form an opening through which the right leg is inserted.

For the pants-like underwear 21 of this embodiment, similar fabric pieces A to the fabric piece A of the first embodiment are used, and is constructed such that the base fabric B is disposed inner side and the membrane C is disposed outer side.

For any of the junction areas 21A-21F, the junction area D1 shown in Fig. 3A is adopted, and the welding is performed from the junction area 21A-21F in that order. It is also possible to adopt the junction area D3 shown in Fig. 3D for any of the junction area 21A-21F and welding is performed. For the junction areas 21C, 21D on the left side and the right side, which are not likely to contact with the menstrual blood or urine leaked out from undergarment, the junction D1 shown in Fig. 3A can be adopted, and sewing that uses a string can be used instead of welding.

At an intersection at which the junction areas intersect, multiple fabric pieces A are overlaid. Therefore, the intersection becomes thick, which may increase rough feeling. In this embodiment, the increase of the rough feeling is restricted by treating junction areas as described below as shown in Fig. 5B. When the junction areas 21A-21F are welded by adopting the junction area D1 shown in Fig. 3A, four fabric pieces A, A, ..., in other words, the left front panel 22, the right front panel 23, the left back panel 24, and the right back panel 25, are joined near the lower edges of the junction area 21A on the front side and the junction area 21B on the back side in such a condition that one corner of each panel butts against each other, and joined by the junction areas 21A, 21B, 21E, and 21F, as shown in Fig. 5B.

These junction areas 21A, 21B, 21E, and 21F are formed in that order.

At first, the left front panel 22 and the right front panel 23 are joined such that respective junction allowances 22a, 23a are folded inward and heat-welded to form the junction area 21A (one junction area).

Then, the left back panel 24 and the right back panel 25 are similarly joined such that respective junction allowances 24a, 25a are folded inward and welded to form the junction area 21B (the other junction area).

Then, the junction area 21E (another junction area) and the junction area 21F (another junction area) are formed continuously. In other words, the left front panel 22 and the right front panel 23 joined as described above and the left back panel 24 and the right back panel 25 similarly joined are joined by welding junction allowances 22c, 24c to form the junction area 21E, and welding junction allowances 23c, 25c to form the junction area 21F.

In this process, the already formed junction area 21B is folded to the left (to the left back panel 24), and an edge portion 21Bb of the junction area 21B is welded to the junction allowance 24c in the junction area 21E. In other words, in appearance, the edge portion 21Bb of the junction area 21B is joined to the junction area 21E to constitute a portion thereof. In contrast, the already formed junction area 21A is folded to the right (to the right front panel 23), and an edge portion 21Aa of the junction area 21A is welded to the junction allowance 23c in the junction area 21F. In other words, in appearance, the edge portion 21Aa of the junction area 21A is joined to the junction area 21F to constitute a portion thereof.

As such, the edge portion 21Aa of the junction area 21A and the edge portion 21Bb of the junction allowance 21B are joined in mutually opposite directions. In other words, since the edge portion 21Aa is joined to the right and the edge portion 21Bb is joined to the left, it is prevented that the edge portions 21Aa, 21Bb are joined in a same direction and the thickness at that portion becomes thick, which increases rough feeling. In other words, if the edge portions 21Aa, 21Bb are joined in a same direction, the thickness in that junction area becomes six times of the thickness of one fabric piece A, whereas the thickness in that junction area can be reduced down to four times of the thickness of one fabric piece A by joining the edge portions 21Aa, 21Bb in mutually opposite directions, and thus the rough feeling can be reduced.

Although in the description above, the edge portion 21Aa of the junction area 21A is faced to the right, and the edge portion 21Bb of the junction area 21B is faced to the left, and then the welding is performed to join them. However, of course, the directions may be vice versa, such that the edge portion 21Aa is faced to the left and the edge portion 21Bb is faced to the right and then welding is performed to join them. In other words, what is required is that the directions of the edge portions 21Aa, 21Bb be opposite each other.

In the pants-like underwear 21 of this embodiment as the waterproof moisture-permeable underwear 10, almost similar advantages to those of the skirt-like underwear 11 of the first embodiment can be achieved.

### <Third embodiment>

An example in which the waterproof moisture-permeable underwear 10 is pants-like underwear 31 is illustrated, with reference to Figs. 6A and 6B. Fig. 6A is a view of the underwear 31 viewed from front and obliquely above, and Fig. 6B is a view taken along an arrowed line B-B in Fig. 6A. The underwear 31 of this embodiment is worn with a higher fit than the underwear 21 of the second embodiment described above. Therefore, the reduction in rough feeling is sought by adopting the junction area D3 shown in Fig. 3D for junction areas 31A-31D as described later.

As shown in Fig. 6A, the underwear 31 is constructed by joining two fabric pieces A, A each cut into a predetermined shape, in other words, a left panel 32 (a front panel and a back panel on the left side formed integrally) and a right panel 33 (a front panel and a back panel on the right side formed integrally) in the junction areas 31A-31D.

The junction area 31A is located at the front center vertically in which a junction allowance 32a on the front of the left panel 32 and a junction allowance 33a on the front of the right panel 33 are joined. In the junction area 31B, a junction allowance 32b on the back of the left panel 32 and a junction allowance 33b on the back of the right panel 33 are joined. The junction area 31A and the junction area 31B continue at their lower edges. The junction area 31C is a junction area of a junction allowance 32c at right front lower edge of the left panel 32 and a junction allowance 32d at right back lower edge of the left panel 32. The junction area 31D is a junction area of a junction allowance 33c at left front lower edge of the right panel 33 and a junction allowance 33d at left back lower edge of the right panel 33. The junction area 31C and the junction area 31D are formed continuously.

By joining as described above, an upper edge 31a of the left panel 32 and the right panel 33 of the underwear 31 is formed annularly, and elastic (not shown) is sewn along the periphery of the upper edge 31a. In addition, lower edges 31b, 31c of the left panel 32 and the right panel 33 are sewn inward to form annular openings through which the left leg or the right leg is inserted respectively.

The pants-like underwear 31 of this embodiment uses similar fabric pieces A to the fabric piece A of the first embodiment, and is constructed such that the base fabric B is disposed inner side and the membrane C is disposed outer side.

Any of the junction areas 31A-31D is welded by adopting the junction area D3 shown in Fig. 3D. It is also possible to adopt the junction area D1 shown in Fig. 3A for any of the junction areas 31A-31D. However, if it is taken into account that the underwear 31 of this embodiment is intended to be worn with a higher fit, it is desirable to adopt the junction area D3 in Fig. 3D, which causes less rough feeling.

In the pants-like underwear 31 of this embodiment as the waterproof moisture-permeable underwear 10, almost similar advantages to those of the skirt-like underwear 11 of the first embodiment can be achieved.

For the junction areas to be welded in the underwear 11, 21, 31 described above in the first to third embodiments, basically the junction area D1 shown in Fig. 3A may be preferably adopted. Since the junction areas in the underwear 11, 21, 31 are folded inward and cannot be seen from outside, the junction areas having good-looking and greater junction strength can be formed. In addition, if less rough feeling is desirable even though junction strength decreases to some extent, the junction area D3 shown in Fig. 3D may be adopted instead.

### <Fourth embodiment>

An example in which the waterproof moisture-permeable liner 40 to which the present invention is applied is the liner 41 of the skirt 42 is illustrated, with reference to Figs. 7A, 7B. Fig. 7A is an oblique view of the skirt 42 viewed from front and obliquely above, and Fig. 7B is an exploded oblique view in which a skirt body 43 and the liner 41 are separated.

The skirt 42 is constructed by the skirt body 43 formed in a roughly truncated cone and acts as an outer fabric, and the liner 41 also formed in a roughly truncated cone and attached inside (behind) the skirt body 43.

The skirt body 43 has four fabric pieces each cut into a predetermined shape, in other words, an upper front panel 44, a lower front panel 45, an upper back panel 46, and a lower back panel 47, and is constructed by joining them in junction areas 43A, 43B, 43C, and 43D. Any of these junction areas 43A-43D is formed by sewing that uses a string. An upper portion of the junction area 43C on the left side is not joined, and a slit is formed therein. Attached to the slit is a zipper 43a that facilitates an easy take-on and off of the skirt 42.

The liner 41 is formed by joining two fabric pieces A, A each cut into a predetermined shape, in other words, a front panel 48 and a back panel 49, in a junction area 41A on the left side and a junction area 41B on the right side. The junction area 41A on the left side is formed by joining respective junction allowances 48a, 49a of the front panel 48 and the back panel 49. The junction area 41A is not formed in a portion that corresponds to the zipper 43a at an upper edge portion. The junction area 41B on the right side is formed by joining respective junction allowances 48b, 49b of the front panel 48 and the back panel 49 from an upper edge 41a to a lower edge 41b of the liner 41.

The liner 41 uses similar fabric pieces A to the fabric piece A of the first embodiment, and is constructed such that the base fabric B is disposed inner side and the membrane C is disposed outer side. Then, the junction areas 41A, 41B are welded by adopting the junction area D2 shown in Fig. 3B. These junction areas 41A, 41B may be joined by sewing that uses a string instead of the welding because these portions are located on the left side and the right side of the skirt 42 and thus the possibility that the menstrual blood or urine leaked out from undergarment worn under the skirt 42 contacts with these portion is quite low.

The liner 41 is attached inside (behind) the skirt body 43 by sewing the annular upper edge 41a to the junction areas 43A, 43B of the skirt body 43, and tying strings 41c, 41c provided to lower edges of the junction areas 41A, 41B on the left and right to respective fastening loops (not shown) correspondingly provided at positions inside (behind) the skirt body 43.

In this embodiment, the upper edge 41a of the liner 41 may be attached to inside the skirt body 43, for example, by using a hook-and-loop fastener, instead of sewing it to the skirt body 43, so that the liner 41 can be easily attached to and detached from the skirt body 43.

In the skirt-like liner 41 of this embodiment as the waterproof moisture-permeable liner 40, almost similar advantages to those of the skirt-like underwear 11 of the first embodiment can be achieved.

### <Fifth embodiment>

An example in which the waterproof moisture-permeable liner 40 to which the present invention is applied is the liner 51 of the short pants 52 is illustrated, with reference to Figs. 8A-8C. Fig. 8A is an oblique view of the short pants 52 viewed from front and obliquely above. Fig. 8B is an oblique view of a liner 51 viewed from front and obliquely above. Fig. 8C is an oblique view of a short pants body 53 viewed from front and obliquely above.

The short pants body 53 has an annular opening at an upper edge 53a through which the wearer' torso is inserted, and also has openings at each of lower edges 53b, 53c through which the left leg or the right leg is inserted respectively. At an upper portion of the front center, a slit is formed, and a zipper 53d that facilitates an easy take-on and off is attached to the slit. Regarding the shape or structure of the short pants body 53, ones similar to those used for general short pants can be used, and thus detailed description on the short pants body 53 is omitted.

The liner 51 is constructed by joining four fabric pieces A, A,..., each cut into a predetermined shape, in other words, a left front panel 54, a right front panel 55, a left back panel 56, and a right back panel 57 in junction areas 51A-51D. The junction area 51A on the front is formed by joining a junction allowance 54a on the right side of the left front panel 54 and a junction allowance 55a on the left side of the right front panel 55. At an upper portion of the junction area 51A that corresponds to the zipper 53d of the short pants body 53, the left front panel 54 and the right front panel 55 are not joined and overlaid each other, and sewn to inside (behind) the short pants body 53. The junction area 51B on the back side is formed by joining a junction allowance 56a on the right side of the left back panel 56 and a junction allowance 57a on the left side of the right back panel 57. The junction area 51C on the left side is formed by joining a junction area 54b on the left side of the left front panel 54 and a junction allowance 56b on the left side of the left back panel 56. Then, the junction area 51D on the right side is formed by joining a junction area 55b on the right side of the right front panel 55 and a junction allowance 57b on the right side of the right back panel 57. By joining as described above, an annular opening through which the wearer' torso is inserted is formed at an upper edge 51a of the liner 51, and openings through which the leg is inserted is formed at a lower edge 51b on the left and at a lower edge 51c on the right, respectively.

The liner 51 uses similar fabric pieces A to the fabric piece A of the first embodiment, and is constructed such that the base fabric B is disposed inner side and the membrane C is disposed outer side. For the junction areas 51A-51D, the junction area D2 shown in Fig. 3B is adopted and the welding is performed. Among these junction areas 51A-51D, the junction area 51C on the left and the junction area 51D on the right are located on the left side and the right side of the short pants 52 and the possibility that the menstrual blood or urine leaked out from undergarment worn inside of the short pants 52 contacts with these portions is quite low, and thus may be joined by sewing that uses a string.

The liner 51 is attached to the short pants body 53 by sewing the annular upper edge 51a to a portion slightly lower than the upper edge 53a in an inner side of (behind) the short pants body 53, and by tying strings 51d, 51d provided to lower edges of the junction area 51C on the left side and the junction area 51D on the right side to respective fastening loops (not shown) correspondingly provided inside the short pants body 53.

In the short pants-like liner 51 of this embodiment as the waterproof moisture-permeable liner 40, almost similar advantages to those of the skirt-like underwear 11 of the first embodiment can be achieved.

The liner 51 described above can be used as a liner of boxer shorts or training wear, for example, made of nylon, to be worn during exercise. The liner 51 has waterproof moisture-permeability, and is lightweight, and has good texture, stretch, breathability, and windproof property. It is therefore quite desirable to be used for a liner for boxer shorts or the like, in which moisture tends to gather because body temperature rises due to exercise. On the surface of the boxer shorts or training wear, any desired colored patterns or characters can be printed readily compared with the fabric piece A. Therefore, boxer shorts or training wear being fashionable and having superior functionalities can be constructed. It is intended that the wearers of boxer shorts or training pants to do strenuous exercise, and thus it is desirable that the liner 51 to be attached inside thereof adopt the junction area D1 shown in Fig. 3A that has a greater junction strength. However, in this case, the junction area D1 is formed inside of the liner 51, in other words, the side seen by others, and is a little inferior in appearance.

Regarding the junction area to be welded in the liners 41, 51 in the fourth and fifth embodiments described above, basically the junction area D2 shown in Fig. 3B is adopted, and the junction area is located outside of the liner and covered by the outer garment and is not exposed to outside (inner side) and thus has good-looking. However, if greater importance is placed on the junction strength, it is desirable to adopt the junction area D1 shown in Fig. 3A that provides a little poor appearance but greater junction strength. Examples of such occasions are boxer shorts or training wear described above.

When junction areas are formed by welding in the waterproof moisture-permeable underwear 10 and the waterproof moisture-permeable liner 40 according to the present invention described above, any of welding described above can be adopted under a condition that required junction strength can be obtained. Which welding is to be adopted may be determined considering other conditions, for example, appearance, ease of processing or the like.

### Description of references in drawings

- 10: waterproof moisture-permeable underwear
- 11: skirt-like underwear
- 11A, 11B: junction area
- 12a, 12b: junction allowance
- 13a, 13b: junction allowance
- 21: pants-like underwear
- 21A-21F: junction area
- 22a, 22b, 22c: junction allowance
- 23a, 23b, 23c: junction allowance
- 24a, 24b, 24c: junction allowance
- 25a, 25b, 25c: junction allowance
- 31: pants-like underwear
- 31A-31D: junction area
- 32a, 32b, 32c: junction allowance
- 33a, 33b, 33c: junction allowance
- 40: waterproof moisture-permeable liner
- 41: liner for a skirt
- 41A-41D: junction area
- 48a, 48b: junction allowance
- 49a, 49b: junction allowance
- 51: liner for short pants
- 51A-51D: junction area
- 54a, 54b: junction allowance
- 55a, 55b: junction allowance
- 56a, 56b: junction allowance
- 57a, 57b: junction allowance
- A: fabric piece
- B: base fabric
- C: membrane
- D1, D2,: D3junction area
- S: seam tape (sealing material)
- S1, S2: the direction of stretch

## Claims

1. A waterproof moisture-permeable underwear (10) to be worn between undergarment and an outer garment worn on the lower body, **characterized in that**:
the waterproof moisture-permeable underwear (10) is constructed by joining, in junction areas (11A,11B), a plurality of two-layered fabric pieces (A), each comprising,
a base fabric (B) disposed such that it faces the undergarment, and
a membrane (C) laminated such that it faces the outer garment and having waterproof moisture-permeability,
wherein a material superior to the membrane (C) in texture is selected for the base fabric (B), and
a material superior to the base fabric (B) in welding property is selected for the membrane (C), and
at least one of the junction areas (11A, 11B) is formed by welding.

2. The waterproof moisture-permeable underwear (10) according to claim 1 **characterized in that** the junction area (D1) formed by welding is formed by welding in such a condition that respective junction allowances (A1) of the fabric pieces (A) being adjacent to each other are folded inward and the membranes (C) in the respective junction allowances(A1) are brought into contact with each other.

3. The waterproof moisture-permeable underwear (10) according to claim 1 **characterized in that** the junction area (D2) formed by welding is formed by welding in such a condition that respective junction allowances (A1) of the fabric pieces (A) being adjacent to each other are extended outward and the base fabrics (B) in the respective junction allowances (A1) are brought into contact with each other.

4. The waterproof moisture-permeable underwear (10) according to claim 1 **characterized in that** the junction area (D2) formed by welding is formed by welding in such a condition that one junction allowance (A1) of respective junction allowances (A1) of the fabric pieces (A) being adjacent to each other is overlaid on the other junction allowance (A1) such that the membrane (C) in the one junction allowance (A1) is brought into contact with the base fabric (B) in the other junction allowance (A1).

5. The waterproof moisture-permeable underwear (10) according to claim 1 **characterized in that** the junction area (21A) formed by welding is formed by welding in such a condition that respective junction allowances (22a,23a) of the fabric pieces (22,23) being adjacent to each other are folded inward and the membranes (C) in the respective junction allowances (22a,23a) are brought into contact with each other, or in such a condition that respective junction allowances (22a,23a) of the fabric pieces (22,23) being adjacent to each other are extended outward and the base fabrics (B) in the respective junction allowances (22a,23a) are brought into contact with each other,
wherein when the fabric pieces (22,23) being joined in the junction area (21 A) are joined in another junction area (21 B) that intersects with these junction areas (21A,21B) in such a condition that one of the junction areas (21A) and the other junction area (21 B) continuously converge each other,
the one junction area (21A) is joined to the another junction area (21B) with its edge portion being folded, and the other junction area (21B) is joined to the another junction area (21A) with its edge portion being folded in a direction opposite to that of the one junction area (21 A).

6. The waterproof moisture-permeable underwear (10) according to claim 1 **characterized in that** the junction area (D3) formed by welding is formed by aligning respective end edges (A2) of the fabric pieces (A) being adjacent to each other, and welding a band of sealing material (S) on the end edges (A2).

7. The waterproof moisture-permeable underwear (10) according to claim 1 **characterized in that** the junction area (21A,21B,21E,21F) of the plurality of fabric pieces (22,23,24,25) to be welded includes a crotch line (22a,23a,24a,25a) and a crotch portion (22c,23c,24c,25c).

8. The waterproof moisture-permeable underwear (10) according to claim 1 **characterized in that** the fabric piece (A) has a degree of stretch in one direction (S1) and a different degree of stretch in another direction (S2) orthogonal to the one direction (S1), and the fabric piece (A) is disposed such that a direction (S1) having a higher degree of stretch is horizontal.

## Patentansprüche

1. Wasserdichte feuchtigkeitsdurchlässige Unterbekleidung (10), die zwischen Unterwäsche und einer äußeren Bekleidung am Unterkörper getragen wird, **dadurch gekennzeichnet, dass**
die wasserdichte feuchtigkeitsdurchlässige Unterbekleidung (10) hergestellt wird durch das Verbinden, in Verbindungsbereichen (11A, 11 B), einer Vielzahl von zweilagigen Stoffteilen (A), die jeweils folgendes umfassen:
einen Basisstoff (B), der so angeordnet ist, dass er der Unterwäsche gegenüberliegt, und
eine Membran (C), die so laminiert ist, dass sie der äußeren Bekleidung gegenüberliegt, und die eine wasserdichte Feuchtigkeitsdurchlässigkeit besitzt,
wobei ein Material mit einer Struktur, die der der Membran (C) überlegen ist, für den Basisstoff (B) gewählt wird, und
ein Material mit einer Schweißeigenschaft, die der des Basisstoffs (B) überlegenen ist, für die Membran (C) gewählt wird, und
wenigstens einer der Verbindungsbereiche (11A, 11B) durch Schweißen gebildet wird.

2. Wasserdichte feuchtigkeitsdurchlässige Unterbekleidung (10) nach Anspruch 1, **dadurch gekennzeichnet, dass** der Verbindungsbereich (D1), der durch Schweißen gebildet ist, durch Schweißen in einem solchen Zustand gebildet wird, dass die jeweiligen Verbindungsabschnitte (A1) der Stoffteile (A), die benachbart zueinander sind, nach innen umgeschlagen sind und sich die Membrane (C) in den jeweiligen Verbindungsabschnitten (A1) berühren.

3. Wasserdichte feuchtigkeitsdurchlässige Unterbekleidung (10) nach Anspruch 1, **dadurch gekennzeichnet, dass** der Verbindungsbereich (D2), der durch Schweißen gebildet ist, durch Schweißen in einem solchen Zustand gebildet wird, dass sich die jeweiligen Verbindungsabschnitte (A1) der Stoffteile (A), die benachbart zueinander sind, nach außen erstrecken und die Basisstoffe (B) in den jeweiligen Verbindungsabschnitten (A1) berühren.

4. Wasserdichte feuchtigkeitsdurchlässige Unterbekleidung (10) nach Anspruch 1, **dadurch gekennzeichnet, dass** der Verbindungsbereich (D2), der durch Schweißen gebildet ist, durch Schweißen in einem solchen Zustand gebildet wird, dass ein Verbindungsabschnitt (A1) der jeweiligen Verbindungsabschnitte (A1) der Stoffteile (A), die benachbart zueinander sind, über dem anderen Verbindungsabschnitt (A1) angeordnet ist, sodass die Membran (C) in dem einen Verbindungsabschnitt (A1) den Basisstoff (B) in dem anderen Verbindungsabschnitt (A1) berührt.

5. Wasserdichte feuchtigkeitsdurchlässige Unterbekleidung (10) nach Anspruch 1, **dadurch gekennzeichnet, dass** der Verbindungsbereich (21A), der durch Schweißen gebildet ist, durch Schweißen in einem solchen Zustand gebildet wird, dass die jeweiligen Verbindungsabschnitte (22a, 23a) der Stoffteile (22, 23), die benachbart zueinander sind, nach innen umgeschlagen sind und sich die Membrane (C) in den jeweiligen Verbindungsabschnitten (22a, 23a) berühren, oder durch Schweißen in einem solchen Zustand gebildet wird, dass sich die jeweiligen Verbindungsabschnitte (22a, 23a) der Stoffteile (22, 23), die benachbart zueinander sind, nach außen erstrecken und sich die Basisstoffe (B) in den jeweiligen Verbindungsabschnitten (22a, 23a) berühren,
wobei, wenn die Stoffteile (22, 23), die in dem Verbindungsbereich (21A) miteinander verbunden sind, in einem weiteren Verbindungsbereich (21 B) verbunden sind, der mit diesen Verbindungsbereichen (21 A, 21 B) in einem solchen Zustand kreuzt, dass einer der Verbindungsbereiche (21 A) und der andere Verbindungsbereich (21 B) kontinuierlich aufeinander zu laufen,
der eine Verbindungsbereich (21A) mit dem anderen Verbindungsbereich (21 B) verbunden ist, wobei sein Randabschnitt umgeschlagen ist, und der andere Verbindungsbereich (21 B) mit dem weiteren Verbindungsbereich (21 A) verbunden ist, wobei sein Randabschnitt in einer Richtung entgegengesetzt zu der des einen Verbindungsbereichs (21A) umgeschlagen ist.

6. Wasserdichte feuchtigkeitsdurchlässige Unterbekleidung (10) nach Anspruch 1, **dadurch gekennzeichnet, dass** der Verbindungsbereich (D3), der durch Schweißen gebildet ist, durch das Ausrichten der jeweiligen Randbereiche (A2) der Stoffteile (A), die benachbart zueinander sind, und das Verschweißen eines Bandes aus Dichtmaterial (S) an den Randbereichen (A2) gebildet wird.

7. Wasserdichte feuchtigkeitsdurchlässige Unterbekleidung (10) nach Anspruch 1, **dadurch gekennzeichnet, dass** der Verbindungsbereich (21A, 21B, 21E, 21 F) der Vielzahl von zu verschweißenden Stoffteilen (22, 23, 24, 25) eine Schrittlinie (22a, 23a, 24a, 25a) und einen Schrittabschnitt (22c, 23c, 24c, 25c) umfasst.

8. Wasserdichte feuchtigkeitsdurchlässige Unterbekleidung (10) nach Anspruch 1, **dadurch gekennzeichnet, dass** das Stoffteil (A) einen Grad der Dehnung in einer Richtung (S1) und einen davon verschiedenen Grad der Dehnung in einer anderen Richtung (S2) senkrecht zu der einen Richtung (S1) aufweist, und das Stoffteil (A) so angeordnet ist, dass eine Richtung (S1), die einen höheren Grad der Dehnung hat, horizontal ist.

## Revendications

1. Sous-vêtement étanche perméable à l'humidité (10) destiné à être porté entre les sous-vêtements et un vêtement de dessus porté sur les membres inférieurs, **caractérisé en ce que** :
le sous-vêtement étanche perméable à l'humidité (10) est fabriqué par l'assemblage, dans les surfaces de jointure (11 A, 11 B), de plusieurs pièces de tissu double-couches (A), comprenant chacune :
un tissu de base (B) disposé de telle sorte qu'il fasse face au sous-vêtement, et
une membrane laminée (C) de telle sorte qu'elle fasse face au vêtement de dessus et soit étanche et perméable à l'humidité,
dans lequel un matériau supérieur en texture à la membrane (C) est choisi pour tissu de la base (B) et
un matériau dont les propriétés de soudure sont supérieures au tissu de la base (B) est choisi pour la membrane (C), et
au moins une des aires de jointure (11 A, 11 B) est formée par soudure.

2. Le sous-vêtement étanche perméable à l'humidité (10) selon la revendication 1 **caractérisé en ce que** la surface de jointure (D1) formée par soudure est formée par soudure de telle manière que les surfaces de jointure respectives (A1) des pièces de tissus (A) adjacentes l'une à l'autre sont repliées vers l'intérieur et les membranes (C) des surfaces de jointure respectives (A1) sont placées en contact l'une avec l'autre.

3. Le sous-vêtement étanche perméable à l'humidité (10) selon la revendication 1 **caractérisé en ce que** la surface de jointure (D2) formée par soudure est formée par soudure de telle manière que les surfaces de jointure respectives (A1) des pièces de tissu (A) adjacentes l'une à l'autre dépassent vers l'extérieur et les tissus de base (B) dans les surfaces de jointure respectives (A1) sont placées en contact l'une avec l'autre.

4. Le sous-vêtement étanche perméable à l'humidité (10) selon la revendication 1 **caractérisé en ce que** la surface de jointure (D2) formée par soudure est formée par soudure de telle manière qu'une surface de jointure (A1) des surfaces de jointure respectives (A1) des pièces de tissu (A) adjacentes l'une à l'autre recouvre l'autre surface de jointure (A1) de telle sorte que la membrane (C) de ladite surface de jointure (A1) est placée en contact avec le tissu de base (B) sur l'autre surface de jointure (A1).

5. Le sous-vêtement étanche perméable à l'humidité (10) selon la revendication 1 **caractérisé en ce que** la surface de jointure (21 A) formée par soudure est formée par soudure de telle manière que les surfaces de jointure respectives (22a, 23a) des pièces de tissu (22, 23) adjacentes l'une à l'autre sont repliées vers l'intérieur et les membranes (C) des surfaces de jointure respectives (22a, 23a) sont placées en contact l'une avec l'autre, ou de telle sorte que les surfaces de jointure (22a, 23a) des pièces de tissu (22, 23) adjacentes l'une à l'autre dépassent vers l'extérieur et les tissus de base (B) des surfaces de jointure respectives (22a, 23a) sont placés en contact l'un avec l'autre,
dans lequel les pièces de tissu (22, 23) jointes dans l'aire de jointure (21 A) sont jointes dans une autre aire de jointure (21 B) en intersection avec lesdites aires de jointure (21 A, 21 B) de telle sorte que l'une des aires de jointure (21 A) et l'autre aire de jointure (21 B) se rejoignent l'une l'autre,
ladite aire de jointure (21 A) est jointe à une autre aire de jointure (21 B) avec la partie comprenant sa bordure repliée, et l'autre aire de jointure (21 B) est jointe à ladite autre aire de jointure (21 A) avec la partie comprenant sa bordure repliée en direction opposée à celle de la première aire de jointure (21 A).

6. Le sous-vêtement étanche perméable à l'humidité (10) selon la revendication 1 **caractérisé en ce que** la surface de jointure (D3) formée par soudure est formée par l'alignement des bordures des extrémités respectives (A2) des pièces de tissu (A) adjacentes l'une à l'autre, et la soudure d'une bande de matériau adhésif (S) aux bordures des extrémités (A2).

7. Le sous-vêtement étanche perméable à l'humidité (10) selon la revendication 1 **caractérisé en ce que** la surface de jointure (21 A, 21 B, 21 E, 21 F) des multiples pièces de tissu (22, 23, 24, 25) à souder comprend une ligne d'entrejambe (22a, 23a, 24a, 25a) et une portion d'entrejambe (22c, 23c, 24c, 25c).

8. Le sous-vêtement étanche perméable à l'humidité (10) selon la revendication 1 **caractérisé en ce que** la pièce de tissu (A) possède un degré d'étirement dans une direction (S1) et un degré différent d'étirement dans une autre direction (S2) perpendiculaire à ladite direction (S1), et la pièce de tissu (A) est disposée de telle sorte qu'une direction (S1) de degré d'étirement supérieur est horizontale.
